(19) Europäisches Patentamt
European Patent Office
Office européen des brevets

(11) Publication number: **0 061 789**
**B1**

(12) **EUROPEAN PATENT SPECIFICATION**

(45) Date of publication of patent specification: **23.10.85**

(21) Application number: **82200267.1**

(22) Date of filing: **03.03.82**

(51) Int. Cl.$^4$: **C 07 D 249/08,**
C 07 D 409/06, A 01 N 43/653
// (C07D409/06, 249:08, 339:02)

(54) **Novel antimicrobial triazole derivatives.**

(30) Priority: **27.03.81 US 248593**
**09.11.81 US 319540**

(43) Date of publication of application:
**06.10.82 Bulletin 82/40**

(45) Publication of the grant of the patent:
**23.10.85 Bulletin 85/43**

(84) Designated Contracting States:
**AT BE CH DE FR GB IT LI NL SE**

(56) References cited:
**EP-A-0 000 017**
**EP-A-0 000 018**
**EP-A-0 000 112**
**EP-A-0 004 917**
**EP-A-0 038 109**
**DE-A-2 724 684**
**DE-A-2 846 127**

(73) Proprietor: **JANSSEN PHARMACEUTICA N.V.**
**Turnhoutsebaan 30**
**B-2340 Beerse (BE)**

(72) Inventor: **Heeres, Jan**
**Leemskuilen 18**
**B-2350 Vosselaar (BE)**
Inventor: **Sturm, Elmar**
**Klusstrasse 66**
**CH-4147 Aesch (CH)**
Inventor: **Backx, Leo J.J.**
**Broekstraat 92**
**B-2370 Arendonk (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

Ketal derivatives of 1-(2-aryl-2-oxoethyl)-1*H*-1,2,4-triazoles are disclosed, inter alia, in the following references:

U.S. Pat. No. 4,079,062;

Brit. Pat. No. 2,026,486; and

Brit. Pat. No. 2,027,701.

Additionally, 1*H*-1,2,4-triazoles having antifungal activity are also described in German Offenlegungschrift 2,724,684 and 2,846,127 and in European Patent Nos. 000.017; 000.018; 000.112; 004.917 and 038.109.

The compounds of the present invention have hitherto neither been disclosed nor suggested in the literature.

DESCRIPTION OF THE PREFERRED EMBODIMENTS

The subject invention relates to a series of novel dithioketal derivatives of 1-(2-oxo-2-phenylethyl)-1*H*-1,2,4-triazoles and the corresponding sulfones and sulfoxides thereof, being represented by the formula

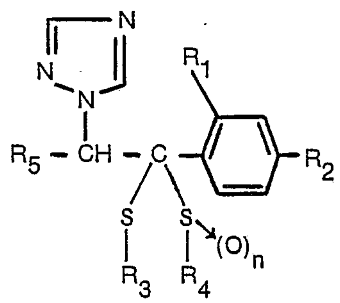

(I),

the possible stereochemically isomeric forms, the acid addition salts and the metal salt complexes thereof, wherein:

$R_1$ and $R_2$ are, each independently, selected from the group consisting of hydrogen, halo and methyl;

$R_3$ and $R_4$ are, each independently, selected from the group consisting of $C_1$—$C_6$-lower alkyl and $C_3$—$C_4$-lower alkenyl, or $R_3$ and $R_4$ form, together with the sulfur atoms to which they are attached and the bridging carbon atom, a 5-, 6- or 7-membered ring which is optionally substituted with 1 to 4 $C_1$—$C_4$-lower alkyl groups;

$R_5$ is a member selected from the group consisting of hydrogen,

$C_1$—$C_4$-lower alkyl, 2-propenyl and 2-propynyl; and n is 0, 1 or 2.

As used in the foregoing and in the following definitions, the term "halo" is generic to fluoro, chloro, bromo and iodo; "lower alkyl" is meant to include straight and branched hydrocarbon radicals having a number of carbon atoms within the indicated limits, such as, for example, methyl, ethyl, propyl, 1-methylethyl, butyl, 1-methylpropyl, 2-methylpropyl and 1,1-dimethylethyl for $C_1$—$C_4$-lower alkyl and, for $C_1$—$C_6$-lower alkyl the foregoing plus the different isomers of pentyl and hexyl.

Preferred compounds within the scope of formula (I) are those wherein $R_1$ is hydrogen or chloro and $R_2$ is chloro.

Particularly preferred are compounds of formula (I) wherein $R_1$ is hydrogen or chloro, $R_2$ is chloro and $R_3$ and $R_4$ together with the sulfur atoms to which they are attached and the bridging carbon atom form a 5 or 6-membered ring which is optionally substituted with a $C_1$—$C_3$-lower alkyl radical.

Especially preferred are compounds of formula (I) wherein $R_1$ is hydrogen or chloro, $R_2$ is chloro, $R_3$ and $R_4$ together with the sulfur atoms to which they are attached and the bridging carbon atom form a 5- or 6-membered ring which is optionally substituted with a $C_1$—$C_3$-lower alkyl radical and $R_5$ is hydrogen or $C_1$—$C_4$-lower alkyl.

More especially preferred are compounds of formula (I) wherein $R_1$ is hydrogen or chloro, $R_2$ is chloro, $R_3$ and $R_4$ together with the sulfur atoms to which they are attached and the bridging carbon atom form a 5- or 6-membered ring which is optionally substituted with a $C_1$—$C_3$ lower alkyl radical and $R_5$ is hydrogen.

Most especially preferred are compounds of formula (I) wherein $R_1$ and $R_2$ are both chloro, $R_3$ and $R_4$ together with the sulfur atoms to which they are attached and the bridging carbon atom form a 5-membered ring which is optionally substituted with a methyl, ethyl or propyl group and $R_5$ is hydrogen.

Preferred species within the scope of formula (I) are the following:

1-[[2-(2,4-dichlorophenyl)-1,3-dithiolan-2-yl]methyl]-1*H*-1,2,4-triazole;

1-[[2-(2,4-dichlorophenyl)-4-methyl-1,3-dithiolan-2-yl]methyl]-1*H*-1,2,4-triazole;

1-[[2-(2,4-dichlorophenyl)-4-ethyl-1,3-dithiolan-2-yl]methyl]-1*H*-1,2,4-triazole;

1-[[2-(2,4-dichlorophenyl)-4-propyl-1,3-dithiolan-2-yl]methyl]-1*H*-1,2,4-triazole;

1-[1-[2-(2,4-dichlorophenyl)-1,3-dithiolan-2-yl]propyl]-1*H*-1,2,4-triazole and;

1-[1-[2-(2,4-dichlorophenyl)-1,3-dithian-2-yl]propyl]-1*H*-1,2,4-triazole.

The compounds of formula (I) wherein n is 0, said compounds being represented by the formula (I-a)

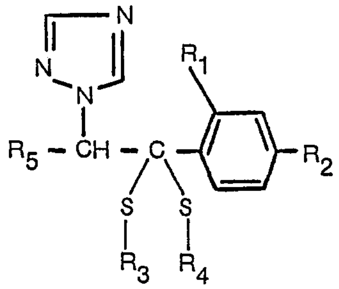

(I—a)

may be prepared by subjecting an appropriate ketone of the formula (II)

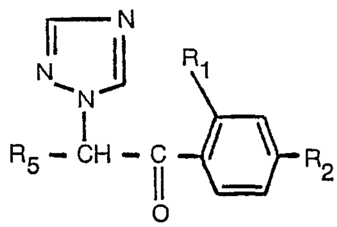

(II)

or an appropriate ketal derivative thereof to a thioketalization reaction with an appropriate thiol of the formula

(III) $R_3$—SH, resp. $R_4$—SH (IV)

or a dithiol of the formula

HS—$R_3$—$R_4$—SH (V)

wherein $R_3$ and $R_4$ are as defined in formula (I).

The said thioketalization reaction is advantageously carried out in the presence of an appropriate Lewis acid, and, unless the acid itself has suitable solvent properties, preferably in an appropriate reaction-inert organic solvent.

Suitable Lewis acids which may be used in the above procedure include strong protonic acids, e.g. sulfonic acids such as methanesulfonic, benzenesulfonic and 4-methylbenzenesulfonic acid, and non-protonic Lewis acids such as, for example, aluminum chloride, zinc chloride, tin chloride and the like.

Appropriate solvents include, for example, aliphatic and aromatic hydrocarbons, e.g. hexane, cyclohexane, heptane, benzene, methylbenzene, dimethylbenzene and the like; chlorinated hydrocarbons such as, for example, di-, tri- and tetrachloromethane, 1,2-dichloroethane, 1,1,2,2-tetrachloroethane, tetrachloroethene and the like; and ethers such as tetrahydrofuran and 1,4-dioxane. In order to enhance the reaction rate, particularly when a protonic acid is used, there may be added to the reaction mixture an appropriate tri(lower alkyloxy)methane, preferably 1,1′,1″-[methylidynetris(oxy)]tris(ethane).

Elevated temperatures are advantageous and, preferably, the reaction is carried out at the reflux temperature of the reaction mixture. Ketal derivatives of the ketones (II) which may be used as starting materials include di-lower alkylketals, such as the dimethyl-, diethyl-, dipropyl- and dibutyl ketals and cyclic ketals such as the dioxolane ketals derived from ethanediol.

The compounds of formula (I) wherein n is 1 or 2, said compounds being represented by the formula (I-b), respectively (I-c), can be derived from the corresponding (I-a) by oxidizing the latter with an appropriate oxidizing agent. By appropriately selecting the oxidizing agent and the reaction circumstances either sulfones or sulfoxides can be obtained substantially free from the other.

(I—b) 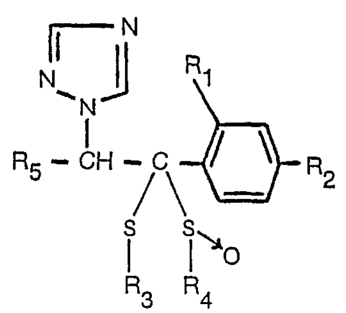

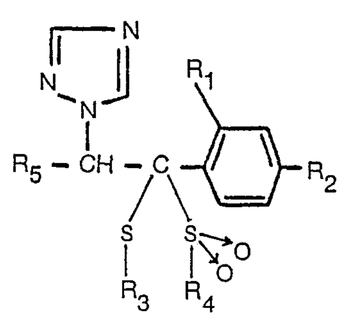 (I—c)

Appropriate oxidizing agents include, for example, periodates, e.g., sodium periodate, potassium

periodate and the like; peroxides, e.g., hydrogen peroxide and the like; and peracids, e.g. perbenzoic acid and preferably 3-chloroperbenzoic acid. These oxidation reactions may be carried out by methodologies which are well-known in the art.

The ketones of formula (II) and the corresponding ketals which are used as starting materials are well-known. Such compounds are described for example in: U.S. Pat. No. 4,079,062; Brit. Pat. No. 2,026,486; Brit. Pat. No. 2,027,701; Brit. Pat. No. 1,533,706; and Brit. Pat. No. 1,464,224.

The thiols of formulas (III), (IV) and (V) are generally known and may all be prepared by the application of art-known methodologies.

It is obvious from formula (I) that the compounds of the present invention may possibly exist under different stereochemically isomeric forms.

Whenever n is the integer 1 or 2 and/or $R_3$ and $R_4$ are not identical or form an alkylene radical which is unsymmetrically substituted, the carbon atom bearing the two sulfur atoms is an asymmetrically substituted carbon atom. Additional chiral centers may exist in $R_3$ and $R_4$ and when $R_5$ is other than hydrogen. Each of these chiral centers may exist in a R- and S-configuration, this R- and S-notation being in correspondance with the rules described by R. S. Cahn, C. Ingold and V. Prelog in Angew. Chem., Int. Ed. Engl., *5*, 385—511 (1966).

Pure stereochemically isomeric forms of the compounds of formula (I) may be obtained by the application of art-known procedures. Diastereoisomers may be separated by physical separation methods such as selective crystallization and chromatography techniques, e.g., counter current distribution, and enantiomers may be separated from each other by the selective crystallization of their diastereoisomeric salts with optically active acids.

Pure stereochemically isomeric forms may also be derived from the corresponding pure stereochemically isomeric forms of the appropriate starting materials, provided that the reaction occurs stereospecifically. Stereochemically isomeric forms of the compounds of formula (I) are naturally intended to be embraced within the scope of the invention.

In view of their basic properties, the compounds of formula (I) may be converted into their acid addition salt forms by reacting them with appropriate acids such as, for example, inorganic acids, e.g., hydrochloric, hydrobromic, hydroiodic, sulfuric, phosphoric, phosphonic, nitric and the like acids, or organic acid, e.g., acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, benzoic, 3-phenyl-2-propenoic, α-hydroxybenzeneacetic, methanesulfonic, ethanesulfonic, hydroxyethanesulfonic, 4-methylbenzenesulfonic, 2-hydroxybenzoic, 4-amino-2-hydroxybenzoic, 2-phenoxybenzoic, 2-acetyloxybenzoic, 2,4-hexadienoic or 1,5-naphthalenedicarboxylic acid.

Metal salt complexes of formula (I) may be obtained by the complexation-reaction of a triazole of formula (I) with an organic or inorganic metal salt such as, for example, hydrohalides, nitrates, sulfates, phosphates, 2,3-dihydroxybutanedioates and the like of copper, manganese, zinc, iron and the like transition metals, which may be present in each of their possible valencies.

Stoichiometrically defined metal salt complexes may be prepared by dissolving a compound of formula (I) in a water-miscible solvent (e.g. warm ethanol, methanol, 1,4-dioxane or *N,N*-dimethylformamide) and adding thereto an aqueous solution of the desired metal salts such as, for example, $CuSO_4.5H_2O$, $Mn(NO_3)_2.4H_2O$, $FeCl_3.6H_2O$ and the like.

The foregoing enumerations are intended to illustrate and not to limit the scope of the present invention.

The compounds of formula (I) and the acid addition salts and metal salt complexes have potent antimicrobial, in particular antifungal, properties and as such they can be used for combating the growth of microorganisms in and/or on living and non-living materials of any nature.

Particularly, the compounds of formula (I) possess a very advantageous antimicrobial spectrum, rendering them useful for the protection of crops without causing undesired side-reactions. Examples of crops within the scope of this invention are the following: cereals, maize, rice, vegetables, sugar-beet, soybeans, ground-nuts, fruit-trees, ornamentals, grapevines, hops, cucurbitaceae (gherkins, cucumbers, melons), solanaceae such as potatoes, tobacco and tomatoes, as well as bananas, cocoa and rubber.

The compounds of formula (I) can be used to reduce or destroy fungal growth on plants of these or related crops or on parts of such plants (e.g., fruits, blossoms, foliage, stams, tubers, roots), whereby the newly outgrowing parts of such plants are also protected against fungal attack. The compounds of this invention are active against phytopathogenic fungi belonging to the following classes: Ascomycetes (e.g. Erysiphaceae, Fusarium, Helminthosporium); Basidiomycetes such as particularly rust-fungi (e.g., Puccinia); Fungi imperfecti (e.g., Moniliales etc., Cercospora and Botrytis) and Oomycetes belonging to the class of the Phycomycetes such as, for example, Phytophthora and Plasmopara. They can further be used as seed-dressings for the treatment of seed (e.g. fruits, tubers, grains) and cuttings to protect them from fungal infection, and against fungi occurring in the soil.

The compounds of formula (I) can be used alone or in admixture with appropriate carriers and/or additives. Appropriate carriers and additives can be solid or fluid and are generally known in the art of formulating, such as, for example, natural and regenerated mineral substances, solvents, dispersants, wetting agents, adhesives, thickeners, binders or fertilizers.

The concentration of the active ingredient in commercial preparations can vary from about 0.1 to about 90%.

For their application the compounds of formula (I) can be formulated in the following composition-forms (whereby suitable concentrations of the active ingredient are indicated within brackets):

solid compositions: dusts
(up to 10%), granulates, coated granulates, impregnated granulates and homogeneous granulates, pellets (from 1 to 80%);

liquid compositions:
a) water-dispersible concentrates:
wettable powders and pastes (25—90% in commercial form, 0.01—15% in the ready for use solution); emulsion- and solution-concentrates (10—50%; 0.01—15% in ready for use solution);
b) solutions (0.1—20%); aerosols.

If desired, in oder to extend their spectrum of activity the compounds of formula (I) may be combined with other appropriate pesticides such as, for example, fungicides, bactericides, insecticides, acaricides, herbicides, plant-growth regulators and the like. Such compositions are intended to be within the scope of the present invention.

The content of active substance in the above agents is from 0.1 to 95%, preferably from 1 to 80%. The forms may be diluted from this concentration down to 0.001%. The employed doses are in general from 0.01 to 10 kg of active substance pro ha, preferably from 0.025 to 5 kg/ha.

The compounds of formula (I) may also be used for the protection of non-living organic materials such as, for example, coatings, e.g., oil paints, dispersion paints, lacquers, whitewash; wood, e.g., timber, lumber, railway sleepers, telephone poles, fences, wood coverings, wicker-work, plywood, particle board, clipboard, joinery, bridges or wood products which are generally used in housebuilding, or pulp wood used in paper manufacture; textiles, e.g., carpets, canvas, awnings, fishing nets, ropes and packing materials; and other organic materials of diverse nature such as, for example, joint fillings of tile walls, tiles in polymeric materials, paper-hangings, hides, leather, artificial leather, bath carpets, shower curtains, technical devices in plastic, glues, mortar as well as walls which are penetrated or fouled by organic materials; cutting oils, etc.

The materials which are treated with agents according to the invention are protected from moulding, rotting, loss of their useful mechanical properties such as breaking strength, resistance to shock and shearing strength; or decay of their optical or other useful properties such as the occurrence of odor, staining, spot formation and dote caused by the following microorganisms: Aspergillus species, Penicillium species, Verticillium species, Alternaria species, Rhizopus species, Mucor species, Paecelomyces species, Saccharomyces species, Trichoderma viride, Chaetomium globosum, Stachybotrys atra, Myrothecium verrucaria, Oospora lactis and other woodrot and wood decay fungi. Special emphasis should be led on the good activity against moulds such as Aspergillus niger, Penicillium funiculosum, Trichoderma viride, Alternaria alternata, fungi such as Chaetomium globosum, Trychophyton mentagrophytes, Coriolus versicolor, Coniophora cerebella, Poria monticola, Merulius (Serpula) lacrymans and Lenzites trabea, and yeasts such as Candida albicans and Saccharomyces species.

The compounds of formula (I) may be used in agents according to the present invention on their own or in combination with appropriate carriers and/or other additives. Appropriate carriers and additives can be solid or liquid and correspond to the substances usually used in formulation techniques such as natural or regenerated inorganic substances, solvents, dispersants, emulsifiers, wetting agents, adhesion agents, thickeners or binding agents.

The compounds of formula (I) show good solubility in organic solvents and in driving gases for aerosols. The lack of color and odor of the compounds of formula (I) is in this connection of great practical value.

The following examples are intended to illustrate and not to limit the scope of the present invention. Unless otherwise indicated all parts therein are by weight.

A. *Examples of chemical preparation*

Example I

A mixture of 6 parts of 4-methylbenzenesulfonic acid and 135 parts of dimethylbenzene is distilled azeotropically to dry. Then there are added 9.5 parts of 1,2-ethanedithiol and 3.5 parts of 1,1′,1″-[methylidynetris(oxy)]trisethane, followed by the addition of 7 parts of 1-[2-(2,4-dichlorophenyl)-2,2-dimethoxyethyl]-1*H*-1,2,4-triazole. The whole is stirred and refluxed for 20 hours with water-separator. The reaction mixture is cooled and stirred with a sodium hydroxide solution 20%. The whole is poured onto water and the layers are separated. The organic phase is washed with a diluted sodium hydroxide solution, dried, filtered and evaporated. The residue is converted into the nitrate salt in 4-methyl-2-pentanone and 2,2′-oxybispropane. The salt is filtered off and crystallized from 2-propanol, yielding 2.7 parts (30%) of 1-[2-(2,4-dichlorophenyl)-1,3-dithiolan-2-ylmethyl]-1*H*-1,2,4-triazole nitrate; m.p. 150.8°C.

Example II

A mixture of 15 parts of 1-[2-(2,4-dichlorophenyl)-1,3-dioxolan-2-ylmethyl]-1*H*-1,2,4-triazole, 61 parts of

1,2-propanedithiol and 17.2 parts of 4-methylbenzenesulfonic acid is stirred and refluxed over week-end. The reaction mixture is cooled, poured onto water and the whole is alkalized with a diluted sodium hydroxide solution. The product is extracted five times with 2,2′-oxybispropane and dichloromethane. The combined extracts are alkalized with a diluted sodium hydroxide solution and the layers are separated. The organic phase is washed with alkaline water and water till neutral, dried, filtered and evaporated. The residue is converted into the nitrate salt in 4-methyl-2-pentanone. The sale is filtered off and dried, yielding 2.5 parts of (*cis+trans*)-1-[2-(2,4-dichlorophenyl)-4-methyl-1,3-dithiolan-2-ylmethyl]-1*H*-1,2,4-triazole mononitrate: m.p. 134.2°C.

Example III

A mixture of 3 parts of 1-(2,4-dichlorophenyl)-2-(1*H*-1,2,4-triazol-1-yl)ethanone and 15 parts of methanesulfonic acid is stirred till all solid enters solution. Then there are added 3 parts of 1,2-butanedithiol and the whole is stirred overnight at room temperature. The reaction mixture is poured onto ice-water and the whole is alkalized with sodium carbonate. The product is extracted with 1,1′-oxybisethane. The extract is washed with water, dried and filtered. The filtrate is saturated with 2-propanol saturated with hydrogen chloride. The formed hydrochloride salt is filtered off and crystallized from a mixture of ethanol and 2,2′-oxybispropane, yielding 2 parts of (*cis-trans*)-1-[[2-(2,4-dichlorophenyl)-4-ethyl-1,3-dithiolan-2-yl]methyl]-1*H*-1,2,4-triazole monohydrochloride; m.p. 171.5—178°C.

Example IV

15 g of 1-(2,4-dichlorophenyl)-2-(1*H*-1,2,4-triazol-1-yl)-ethanone (prepared by acid hydrolysis of 1-[[2-(2,4-dichlorophenyl)-1,3-dioxolan-2-yl]methyl]-1*H*-1,2,4-triazole) and 7 parts of 1,3-propanedithiol were dissolved in 100 ml of 1,2-dichloroethane. 11.5 parts of aluminum chloride was added in small portions with stirring. After the addition the reaction mixture was refluxed for 3 hours, cooled and poured onto ice-water. Extraction with 100 ml of dichloromethane yielded 15 g of a pale yellow oil which was purified by chromatography over a silica column using dichloromethane as eluent. There was obtained 11 g of 1-[[2-(2,4-dichlorophenyl)-1,3-dithian-2-yl]methyl]-1*H*-1,2,4-triazole as an oil.

Example V

A solution of 4 g of 1-[[2-(2,4-dichlorophenyl)-1,3-dithiolan-2-yl]methyl]-1*H*-1,2,4-triazole in 100 ml of dichloromethane was cooled to 0°C and a solution of 2.3 g of 3-chloroperbenzoic acid in 25 ml of dichloromethane was added in the course of 20 min. After the addition the mixture was stirred for 3 hours at room temperature. The solution was filtered, shaken with dilute sodium bisulfite solution then with bicarbonate, dried and evaporated. The resulting oil was not quite pure by TLC and was chromatographed over alumina using dichloromethane-methanol (20:1) as eluent. The main fraction yielded 2.5 g of 1-[[2-(2,4-dichlorophenyl)-1-oxo-1,3-dithiolan-2-yl]methyl]-1*H*-1,2,4-triazole; m.p. 40—60°C.

The compounds prepared according to Examples 1 to 4 or prepared in an analogous manner are listed in the following Table I.

TABLE I

Compounds of formula I

| No. | $R_1$ | $R_2$ | $R_3$, $R_4$ | n | $R_5$ | Salt form | Phys. data |
|---|---|---|---|---|---|---|---|
| 1 | Cl | Cl | $-CH_2-CH_2-$ | 0 | H | $HNO_3$ | mp. 150.8°C |
| 2 | Cl | Cl | $-CH_2-CH_2-$ | 0 | H | base | mp. 91–93°C<br>NMR – (see below) |
| 3 | H | Cl | $-CH_2-CH_2-$ | 0 | H | base | mp. 88–92°C |
| 4 | $CH_3$ | Br | $-CH_2-CH_2-$ | 0 | H | – | – |
| 5 | H | $CH_3$ | $-CH_2-CH_2-$ | 0 | H | – | – |
| 6 | Br | Br | $-CH_2-CH_2-$ | 0 | H | – | – |
| 7 | Cl | Cl | $-CH_2-CH(CH_3)-$ | 0 | H | cis+trans,<br>$HNO_3$ | mp. 134.2°C |
| 8 | Cl | Cl | $-CH_2-CH(CH_3)-$ | 0 | H | base | oil, NMR –<br>(see below) |
| 9 | Cl | Cl | $-CH_2-CH(C_2H_5)-$ | 0 | H | cis+trans,<br>HCl | mp. 171.5–178.0° C |
| 10 | Cl | Cl | $-CH_2-CH(C_3H_7)-$ | 0 | H | cis+trans,<br>HCl | mp. 133–158°C |
| 11 | Cl | Cl | $-CH_2-CH(C_4H_9)-$ | 0 | H | – | – |
| 12 | Cl | Cl | $-CH(CH_3)-CH(CH_3)-$ | 0 | H | – | – |
| 13 | Cl | Cl | $-CH(CH_3)-CH(C_2H_5)$ | 0 | H | – | – |
| 14 | Cl | Cl | $-CH_2-CH_2-CH_2-$ | 0 | H | base | viscous oil.<br>NMR – (see below) |
| 15 | Cl | Cl | $-CH_2-CH_2-CH-$<br>\|<br>$CH_3$ | 0 | H | – | – |
| 16 | Cl | Cl | $-CH-CH_2-CH-$<br>\| \|<br>$CH_3$ $CH_3$ | 0 | H | – | – |
| 17 | Cl | Cl | $CH_3$<br>\|<br>$-CH_2-C-CH_2-$<br>\|<br>$CH_3$ | 0 | H | – | – |
| 18 | Cl | Cl | $-(CH_2)_4-$ | 0 | H | – | mp. 80–82°C |
| 19 | Cl | Cl | $-CH_3$, $-CH_3$ | 0 | H | – | – |
| 20 | Cl | Cl | $-C_2H_5$, $-C_2H_5$ | 0 | H | – | – |

TABLE I (Continued)

| No. | $R_1$ | $R_2$ | $R_3$, $R_4$ | n | $R_5$ | Salt form | Phys. data |
|---|---|---|---|---|---|---|---|
| 21 | Cl | Cl | $-C_3H_7$, $-C_3H_7$ | 0 | H | – | – |
| 22 | Cl | Cl | $-C_4H_9$, $-C_4H_9$ | 0 | H | – | – |
| 23 | Cl | Cl | $-CH_2-CH{=}CH_2$, $-CH_2-CH{=}CH_2$ | 0 | H | – | – |
| 24 | Cl | Cl | $-CH_2-CH_2-$ | 1 | H | base | mp. 40–60°C NMR – (see below) |
| 25 | Cl | Cl | $-CH_2-CH_2-$ | 2 | H | – | mp. 139–141°C |
| 26 | Cl | Cl | $-CH_2-CH(CH_3)-$ | 1 | H | – | – |
| 27 | Cl | Cl | $-CH_2-CH(C_2H_5)-$ | 1 | H | – | – |
| 28 | Cl | Cl | $-CH_2-CH(C_3H_7)-$ | 1 | H | – | – |
| 29 | Cl | Cl | $-(CH_2)_3-$ | 1 | H | – | – |
| 30 | Cl | Cl | $-CH_2-CH_2-$ | 0 | $C_2H_5$ base | | mp. 117–119°C |
| 31 | Cl | Cl | $-CH_2-CH_2-CH_2-$ | 0 | $C_2H_5$ base | | viscous oil |

NMR-data: (CDCl , δ in ppm)

Compound 2: δ = 6.9—7.9 (m, 5H, phenyl and triazole protons)
= 5.1 (s, 2H, methylene group)
= 3.22 (s, 4H, dithiolane protons)

Compound 8: δ = 7.0—8.0 (m, 5H, phenyl and triazole protons)
= 5.2 (d, 2H, methylene protons)
= 2.7—4.1 (m, 3H, dithiolane protons)
= 1.25—1.5 (q, 3H, methyl) (cis-trans mixture)

Compound 14: δ = 7.15—7.95 (m, 5H, phenyl and triazole protons)
= 5.2 (s, 2H, methylene group)
= 2.7—3.1 (m, 4H, dithiane ring)
= 1.7—2.2 (m, 2H, dithiane ring)

Compound 24: δ = 8.2 (2, 1H, triazole)
= 7.7 (s, 1H, triazole)
= 7.1—7.5 (m, 3H, phenyl protons)
= 5.0—5.75 (q, 2H, methylene protons)
= 2.5—4.2 (m, 4H, dithiolane ring protons).

Example VI

Activity against Puccinia graminis on wheat.

*Residual protective action:*

Wheat plants were sprayed 6 days after sowing with a spray broth (0.06% of active substance) prepared from a wettable powder of the active substance. After 24 hours the treated plants were infected with a suspension of Uredospores of the fungus. After an incubation period of 48 hours at 95—100% relative humidity and at about 20°C the plants were stood in a greenhouse at approx. 22°C. The development of rust-pustules was evaluated 12 days after the infection.

*Systemic action:*

5 Days after sowing wheat plants are sprayed with a spray broth (containing 0.006% of active substance; the amount of the spray being proportional with the soil-volume) prepared from a wettable powder of the active substance. After 48 hours the treated plants are infected with a suspension of Uredospores of the fungus. After an incubation period of 48 hours at 95—100% relative humidity and at 20°C the treated plants are stood in a glass-house at about 22°C. The rust-pustules are evaluated 12 days after the day of infection.

Example VII

Activity against Cercospora arachidicola on ground-nut plants.

Ground-nut plants, between 10 cm and 15 cm in height, are sprayed with a spray broth (containing 0.02% of active substance) prepared from a wettable powder of the active substance. After about 48 hours the treated plants are infected by dusting them with a suspension of conidia of the fungus. The infected plants are then incubated for about 72 hours at 21°C and at high relative humidity and then stood in the glass-house. Fungal infection is evaluated 12 days after the day of infection on basis of the number and the extend of the appearing spots.

Example VIII

Activity against Erysiphe graminis on barley.

*Residual protective action:*

Barley plants, about 8 cm in height, are sprayed with a spray broth (containing 0.02% of active substance) prepared from a wettable powder of the active substance. After 3—4 hours the treated plants are dusted with conidia of the fungus. The infected barley plants are then placed in a glass-house at about 22°C and fungal attack is evaluated. 10 days after the day of infection.

*Systemic action:*

A spray broth (containing 0.006% of the active substance; the amount being proportional with the soil-volume), prepared from a wettable powder of the active substance is administered to barley plants, about 8 cm in height, while care is taken that the external parts of the plants do not enter into contact with the spray. After 48 hours the treated plants are dusted with conidia of the fungus. The infected barley-plants are stood in a glass-house at 22°C and the fungal infection is evaluated after 10 days.

Example IX

Activity against Botrytis cinerea on fruit (apples).

Artificially damaged apples were treated by dropping a spray, prepared from a wettable powder of the active ingredient, on the damaged area. The treated fruits are subsequently inoculated with a spore suspension of Botrytis cinerea and incubated for a week at about 20°C and at high air humidity. Evaluation is performed by counting the moulded damaged areas and deriving therefrom the fungicidal activity of the test substances.

The results obtained in the test procedures of Examples VI, VII, VIII and IX are given in the following table, wherein the meaning of the indicated scores is as follows

| score | % disease control |
|---|---|
| 1 | >95% |
| 3 | 80—90% |
| 6 | 50—80% |
| 9 | <50% |

TABLE II

Activity scores of compounds (I) in anti-fungal tests

| Test / Compound | P. graminis | | C. arachidicola | E. graminis | | B. cinerea |
|---|---|---|---|---|---|---|
| | foliar 200 ppm | soil 60 ppm | foliar 200 ppm | foliar 200 ppm | soil 60 ppm | 200 ppm |
| 3 | 1 | 3 | 1 | 6 | 9 | 9 |
| 14 | 1 | 1 | 1 | 1 | 6 | 1 |
| 8 | 1 | 1 | 1 | 1 | 1 | 1 |
| 2 | 1 | 1 | 1 | 1 | 6 | 1 |
| 10 | – | – | – | 1 | 3 | – |
| 30 | 1 | 1 | 1 | 1 | 1 | 3 |
| 31 | 1 | 9 | 1 | 1 | 9 | 9 |

Example X

In-vitro antifungal screening.

The subject compounds are tested against the following fungi:

1. Coriolus versicolor (white rot) (C.v.)
2. Coniophora cerebella (brown rot) (C.c.)
3. Poria monticola (brown rot) (P.m.)
4. Chaetonium globosum (C.g.)
5. Pullularia pullulans (blue stain) (P.p.)
6. Aspergillus candidus (A.c.)
7. Penicillium islandicum (P.i.)
8. Cladosporium resinae (C.r.)
9. Aspergillus niger (A.n.)
10. Aspergillus flavus (A.f.)
11. Trichoderma viride (T.v.)
12. Mucor sp. (Muc.)
13. Rhizopus sp. (Rh.)
14. Absidia ramosa (A.r.)

The fungi are grown on malt agar at 25°C and 7 days old cultures are used in the tests. 10 mg of each test substance is dissolved first in 5 ml of ethanol/water l/l and the thus obtained stock solutions are diluted with water in such a way that the final concentration in the Petri-dish after mixing with the warm agar is 10, 1 or 0.1 ppm.

The agar is inoculated with ±1 $mm^3$ mycelium in the centre of the Petri-dish and incubated at 25°C. The results are evaluated after 14 days by measuring the diameter of fungal growth in mm. and expressed according to the following score system.

| score | growth in % of control |
|---|---|
| 0 | none |
| 1 | ⩽25% |
| 2 | 25—50% |
| 3 | 50—75% |
| 4 | >75% |

The results are given in the following tables III-a and III-b.

TABLE III–a

Scores in in-vitro antifungal screening

| dose in ppm / compound | C.v. | | | C.c. | | | P.m. | | | C.g. | | | P.p. | | | A.c. | | | P.i. | | |
|---|---|---|---|---|---|---|---|---|---|---|---|---|---|---|---|---|---|---|---|---|---|
| | 10 | 1 | 0.1 | 10 | 1 | 0.1 | 10 | 1 | 0.1 | 10 | 1 | 0.1 | 10 | 1 | 0.1 | 10 | 1 | 0.1 | 10 | 1 | 0.1 |
| 1 | 0 | 3 | – | 1 | 4 | – | 0 | 4 | – | – | – | – | 0 | 2 | – | 0 | 4 | – | 0 | 2 | – |
| 7 | 0 | 0 | 4 | 0 | 1 | 4 | 0 | 1 | 4 | 0 | 2 | – | 0 | 1 | 4 | 0 | 4 | 4 | 0 | 1 | 3 |
| 9 | 0 | 2 | – | 0 | 0 | – | – | – | – | 0 | 2 | – | 0 | 1 | – | 2 | 4 | – | 1 | 2 | – |
| 10 | 1 | 4 | – | 0 | 0 | – | | | | 0 | 2 | – | 1 | 1 | – | – | – | – | 0 | 2 | – |

TABLE III–b

Scores in in-vitro antifungal screening

| dose in ppm / compound | C.r. | | | A.n. | | | A.f. | | | T.v. | | | Muc. | | | Rh. | | | A.r. | | |
|---|---|---|---|---|---|---|---|---|---|---|---|---|---|---|---|---|---|---|---|---|---|
| | 10 | 1 | 0.1 | 10 | 1 | 0.1 | 10 | 1 | 0.1 | 10 | 1 | 0.1 | 10 | 1 | 0.1 | 10 | 1 | 0.1 | 10 | 1 | 0.1 |
| 1 | 0 | 0 | – | 0 | 4 | – | 2 | 3 | – | 4 | 4 | – | – | – | – | – | – | – | – | – | – |
| 7 | 0 | 0 | 4 | 0 | 4 | 4 | 3 | 3 | 4 | 4 | 4 | – | 4 | 4 | – | 2 | 4 | – | 4 | 4 | – |
| 9 | 0 | 1 | – | 2 | 4 | – | 1 | 2 | – | 4 | 4 | – | 3 | 4 | – | 1 | 4 | – | 1 | 3 | – |
| 10 | 0 | 3 | – | 2 | 4 | – | 2 | 3 | – | 4 | 4 | – | 1 | 4 | – | 1 | 4 | – | 1 | 4 | – |

B. Formulation Examples.

Example XI

*Dusts:* The following substances are used to prepare
a) 5% and b) a 2% dust:

a) 5 parts of active substance
95 parts of talc;

b) 2 parts of active substance
1 part of highly dispersed silicic acid
97 parts of talc.

The active substances are mixed with the carriers and ground and in this form can be processed to dusts for application.

Example XII

*Granulate:* The following substances are used to prepare a 5% granulate:

5 parts of active substance
0.25 parts of epichlorohydrin
0.25 parts of cetyl polyglycol ether
3.25 parts of polyethylene glycol
91 parts of kaolin (particle size 0.3—0.8 mm).

The active substance is mixed with epichlorohydrin and the mixture is dissolved in 6 parts of 2-propanone. Then polyethylene glycol and cetyl polyglycol ether are added. The resultant solution is sprayed on kaolin and the 2-propanone is evaporated in vacuo. Such a micro-granulate is advantageously used for combating soil fungi.

Example XIII

*Wettable powders:* The following constituents are used to prepare
a) a 70%, b) a 40%, c) and d) a 25% and e) a 10% wettable powder:

a) 70 parts of active substance
5 parts of sodium dibutylnaphthylsulfonate
3 parts of naphthalenesulfonic acid/phenolsulfonic acid/formaldehyde condensate (3:2:1)
10 parts of kaolin
.12 parts of Champagne chalk.

b) 40 parts of active substance
5 parts of sodium ligninsulfonate
1 part of sodium dibutylnaphthalenesulfonic acid
54 parts of silicic acid.

c) 25 parts of active substance
4.5 parts of calcium ligninsulfonate
1.9 parts of Champagne chalk/hydroxyethyl cellulose mixture (1:1)
1.5 parts of sodium dibutylnaphthalenesulfonate
19.5 parts of silicic acid
19.5 parts of Champagne chalk
28.1 parts of kaolin

d) 25 parts of active substance
2.5 parts of isooctylphenoxy-polyethylene-ethanol
1.7 parts of a Champagne chalk/hydroxyethyl cellulose mixture (1:1)
8.3 parts of sodium aluminium silicate
16.5 parts of kieselguhr
46 parts of kaolin.

e) 10 parts of active substance
3 parts of a mixture of the sodium salts of saturated fatty alcohol sulfates
5 parts of naphthalenesulfonic acid/formaldehyde condensate
82 parts of kaolin.

The active substances are intimately mixed in suitable mixers with the additives and ground in appropriate mills and rollers. Wettable powders of excellent wettability and suspension powder are obtained. These wettable powders can be diluted with water to give suspensions of the desired concentration and can be used in particular for leaf application.

Example XIV

*Emulsifiable concentrates:* the following substances are used to prepare a 25% emulsifiable concentrate:

25 parts of active substance
2.5 parts of epoxidised vegetable oil
10 parts of an alkylarylsulfonate/fatty alcohol polyglycol ether mixture
5 parts of dimethyl formamide
57.5 parts of dimethylbenzene.

By diluting such a concentrate with water it is possible to prepare emulsions of the desired concentration, which are especially suitable for leaf application.

**Claims**

1. A compound selected from the group consisting of a triazole derivative having the formula

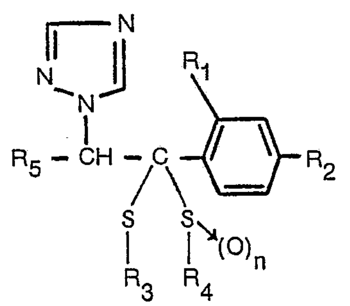

(I),

the possible stereochemically isomeric forms, the acid addition salts and the metal salt complexes thereof, wherein:

$R_1$ and $R_2$ are, each independently, selected from the group consisting of hydrogen, halo and methyl;

$R_3$ and $R_4$ are, each independently, selected from the group consisting of $C_1$—$C_6$-lower alkyl and $C_3$—$C_4$-lower alkenyl, or $R_3$ and $R_4$ form, together with the sulfur atoms to which they are attached and the bridging carbon atom, a 5-, 6- or 7-membered ring which is optionally substituted with 1 to 4 $C_1$—$C_4$-lower alkyl groups;

$R_5$ is a member selected from the group consisting of hydrogen, $C_1$—$C_4$-lower alkyl, 2-propenyl and 2-propynyl; and n is 0, 1 or 2.

2. A compound according to claim 1 wherein $R_1$ is hydrogen or chloro and $R_2$ is chloro.

3. A compound according to claim 1 wherein $R_1$ is hydrogen or chloro, $R_2$ is chloro, $R_3$ and $R_4$ together with the sulfur atoms to which they are attached and the bridging carbon atom form a 5- or 6-membered ring which is optionally substituted with a $C_1$—$C_3$-lower alkyl radical and $R_5$ is hydrogen or $C_1$—$C_4$-lower alkyl.

4. A compound according to claim 1 wherein $R_1$ and $R_2$ are both chloro, $R_3$ and $R_4$ together with the sulfur atoms to which they are attached and the bridging carbon atom form a 5-membered ring which is optionally substituted with a methyl, ethyl or propyl group and $R_5$ is hydrogen.

5. A compound selected from the group consisting of 1-[[2-(2,4-dichlorophenyl)-1,3-dithiolan-2-yl]methyl]-1*H*-1,2,4-triazole and the acid addition salts and metal salt complexes thereof.

6. A compound selected from the group consisting of 1-[[2-(2,4-dichlorophenyl)-4-methyl-1,3-dithiolan-2-yl]methyl]-1*H*-1,2,4-triazole and the acid addition salts and metal salt complexes thereof.

7. A compound selected from the group consisting of 1-[[2-(2,4-dichlorophenyl)-4-ethyl-1,3-dithiolan-2-yl]methyl]-1*H*-1,2,4-triazole and the acid addition salts and metal salt complexes thereof.

8. A compound selected from the group consisting of 1-[[2-(2,4-dichlorophenyl)-4-propyl-1,3-dithiolan-2-yl]methyl]-1*H*-1,2,4-triazole and the acid addition salts and metal salt complexes thereof.

9. A compound selected from the group consisting of 1-[1-[2-(2,4-dichlorophenyl)-1,3-dithiolan-2-yl]propyl]-1*H*-1,2,4-triazole and the acid addition salts and metal salt complexes thereof.

10. A compound selected from the group consisting of 1-[1-[2-(2,4-dichlorophenyl)-1,3-dithian-2-yl]propyl]-1*H*-1,2,4-triazole and the acid addition salts and metal salt complexes thereof.

11. An antifungal composition, comprising inert carrier materials and as an active ingredient an antifungally effective amount of a compound according to any one of claims 1 to 10.

12. A compound according to any one of claims 1 to 10 or a composition according to claim 11 for use as an antifungal agent.

13. A method of combating fungi which comprises contacting said fungi with an effective antifungal amount of a compound according to any one of claims 1 to 10.

14. A process of preparing a compound according to claim 1, characterized in that a ketone of the formula

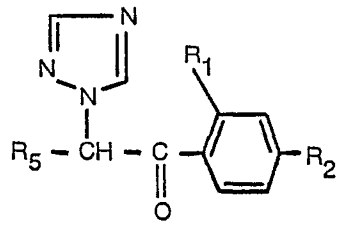

(II)

wherein $R_1$, $R_2$ and $R_5$ are as defined in claim 1, or a ketal derivative thereof, is subjected to a thioketalization reaction with a thiol of the formula

(III) $R_3$—SH or $R_4$—SH (IV)

or a dithiol of the formula

HS—$R_3$—$R_4$—SH (V)

wherein $R_3$ and $R_4$ are as defined in claim 1 in order to obtain a compound of the formula

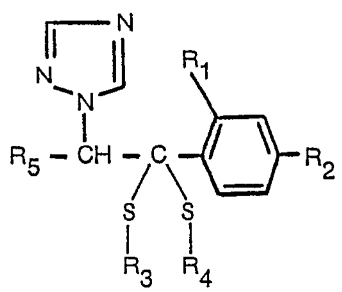

(I—a)

and optionally oxidizing the thus obtained compound (I-a) to obtain a compound of the formula

(I—b) 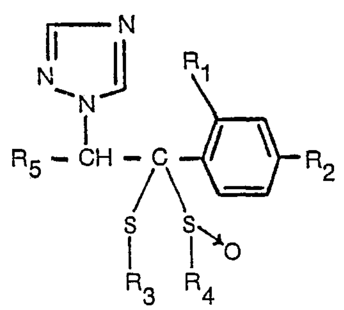 or 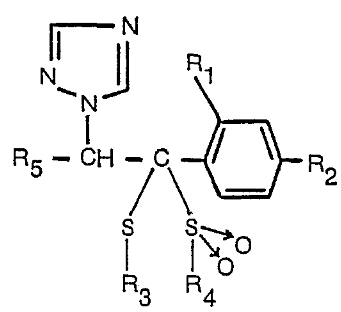 (I—c)

and, if desired, preparing acid addition salts or metal salt complexes of the products of the above steps, and also, if desired, preparing stereochemically isomeric forms of compound (I).

15. A process of preparing a compound selected from the group consisting of 1-[2-(2,4-dichlorophenyl)-1,3-dithiolan-2-ylmethyl]-1*H*-1,2,4-triazole and the acid addition salts and metal salt complexes thereof, characterized by the thioketalization of 1-(2,4-dichlorophenyl)-2-(1*H*-1,2,4-triazol-1-yl)ethanone or a ketal derivative thereof with 1,2-ethanedithiol.

16. A process of preparing a compound selected from the group consisting of 1-[2-(2,4-dichlorophenyl)-4-methyl-1,3-dithiolan-2-ylmethyl]-1*H*-1,2,4-triazole and the stereochemically isomeric forms and acid addition salts and metal salt complexes thereof, characterized by the thioketalization of 1-(2,4-dichlorophenyl)-2-(1*H*-1,2,4-triazol-1-yl)ethanone or a ketal derivative thereof with 1,2-propanedithiol.

17. A process of preparing a compound selected from the group consisting of 1-[[2-(2,4-dichlorophenyl)-4-ethyl-1,3-dithiolan-2-yl]methyl]-1*H*-1,2,4-triazole and the stereochemically isomeric forms and acid addition salts and metal salt complexes thereof, characterized by the thioketalization of 1-(2,4-dichlorophenyl)-2-(1*H*-1,2,4-triazol-1-yl)ethanone or a ketal derivative thereof with 1,2-butanedithiol.

18. A process of preparing a compound selected from the group consisting of 1-[[2-(2,4-dichlorophenyl)-4-propyl-1,3-dithiolan-2-yl]methyl]-1*H*-1,2,4-triazole and the stereochemically isomeric forms and acid addition salts and metal salt complexes thereof, characterized by the thioketalization of 1-(2,4-dichlorophenyl)-2-(1*H*-1,2,4-triazol-1-yl)ethanone or a ketal derivative thereof with 1,2-pentanedithiol.

**Patentansprüche**

1. Eine Verbindung, ausgewählt aus der Gruppe, die aus einem Triazolderivat mit der Formel

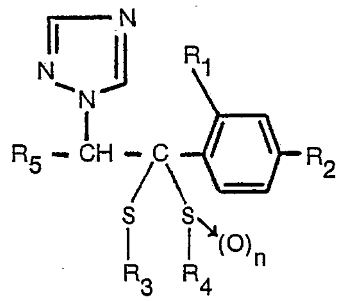

(I),

den möglichen stereochemischen isomeren Formen, den Säureadditionssalzen und den Metallsalzkomplexen hievon besteht, worin:

$R_1$ und $R_2$ jeweils unabhängig ausgewählt sind aus der aus Wasserstoff, Halogen und Methyl bestehenden Gruppe;

$R_3$ und $R_4$ jeweils unabhängig ausgewählt sind aus der aus $C_1$—$C_6$-Niederalkyl und $C_3$—$C_4$-Niederalkenyl bestehenden Gruppe oder $R_3$ und $R_4$, gemeinsam mit den Schwefelatomen, an die sie gebunden sind, und mit dem Kohlenstoffbrückenatom einen 5-, 6- oder 7-gliedrigen Ring bilden, der gegebenenfalls mit 1 bis 4 $C_1$—$C_4$-Niederalkylgruppen substituiert ist;

$R_5$ ein aus der aus Wasserstoff, $C_1$—$C_4$-Niederalkyl, 2-Propenyl und 2-Propinyl bestehenden Gruppe ausgewähltes Glied ist; und

n den Wert 0, 1 oder 2 aufweist.

2. Eine Verbindung nach Anspruch 1, worin $R_1$ Wasserstoff oder Chlor bedeutet und $R_2$ Chlor darstellt.

3. Eine Verbindung nach Anspruch 1, worin $R_1$ Wasserstoff oder Chlor bedeutet, $R_2$ Chlor darstellt, $R_3$ und $R_4$ gemeinsam mit den Schwefelatomen, an die sie gebunden sind, und mit dem Kohlenstoffbrückenatom einen 5- oder 6-gliedrigen Ring bilden, der gegebenenfalls mit einem $C_1$—$C_3$-Niederalkylrest substituiert ist, und $R_5$ Wasserstoff oder $C_1$—$C_4$-Niederalkyl bedeutet.

4. Eine Verbindung nach Anspruch 1, worin $R_1$ und $R_2$ jeweils Chlor bedeuten, $R_3$ und $R_4$ zusammen mit den Schwefelatomen, an die sie gebunden sind, und mit dem Kohlenstoffbrückenatom einen 5-gliedrigen Ring bilden, der gegebenenfalls mit einer Methyl-, Ethyl- oder Propylgruppe substituiert ist, und $R_5$ Wasserstoff bedeutet.

5. Eine Verbindung, ausgewählt aus der aus 1-[[2-(2,4-Dichlorphenyl)-1,3-dithiolan-2-yl]methyl]-1*H*-1,2,4-triazol und den Säureadditionssalzen und Metallsalzkomplexen hievon bestehenden Gruppe.

6. Eine Verbindung, ausgewählt aus der aus 1-[[2-(2,4-Dichlorphenyl)-4-methyl-1,3-dithiolan-2-yl]methyl]-1*H*-1,2,4-triazol und den Säureadditionssalzen und Metallsalzkomplexen hievon bestehenden Gruppe.

7. Eine Verbindung, ausgewählt aus der aus 1-[[2-(2,4-Dichlorphenyl)-4-ethyl-1,3-dithiolan-2-yl]methyl]-1*H*-1,2,4-triazol und den Säureadditionssalzen und Metallsalzkomplexen hievon bestehenden Gruppe.

8. Eine Verbindung, ausgewählt aus der aus 1-[[2-(2,4-Dichlorphenyl)-4-propyl-1,3-dithiolan-2-yl]methyl]-1*H*-1,2,4-triazol und den Säureadditionssalzen und Metallsalzkomplexen hievon bestehenden Gruppe.

9. Eine Verbindung, ausgewählt aus der aus 1-[1-[2-(2,4-Dichlorphenyl)-1,3-dithiolan-2-yl]propyl]-1*H*-1,2,4-triazol und den Säureadditionssalzen und Metallsalzkomplexen hievon bestehenden Gruppe.

10. Eine Verbindung, ausgewählt aus der aus 1-[1-[2-(2,4-Dichlorphenyl)-1,3-dithian-2-yl]propyl]-1*H*-1,2,4-triazol und den Säureadditionssalzen und Metallsalzkomplexen hievon bestehenden Gruppe.

11. Eine antifungale Zusammensetzung, umfassend inerte Trägermaterialien und als einen wirksamen Bestandteil eine antifungal wirksame Menge einer Verbindung gemäß einem der Ansprüche 1 bis 10.

12. Eine Verbindung nach einem der Ansprüche 1 bis 10 oder eine Zusammensetzung nach Anspruch 11 zur Anwendung als ein antifungales Mittel.

13. Eine Verfahren zur Bekämpfung von Pilzen, welches ein Inberührungbringen dieser Pilze mit einer wirksamen antifungalen Menge einer Verbindung gemäß einem der Ansprüche 1 bis 10 umfaßt.

14. Ein Verfahren zur Herstellung einer Verbindung gemäß Anspruch 1, dadurch gekennzeichnet, daß ein Keton der Formel

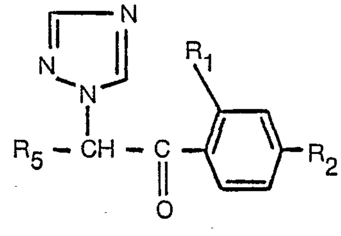

(II)

worin $R_1$, $R_2$ und $R_3$ wie in Anspruch 1 definiert sind, oder ein Ketalderivat hievon einer Thioketalisierungsreaktion mit einem Thiol der Formel

(III) $R_3—SH$ oder $R_4—SH$ (IV)

oder mit einem Dithiol der Formel

$HS—R_3—R_4—SH$ (V)

worin $R_3$ und $R_4$ wie in Anspruch 1 definiert sind, zur Ausbildung einer Verbindung der Formel

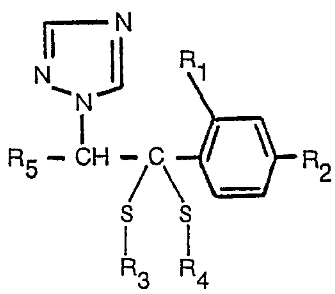

(I—a)

unterworfen wird und gegebenenfalls die solcherart erhaltene Verbindung (I-a) zu einer Verbindung der Formel

(I—b) 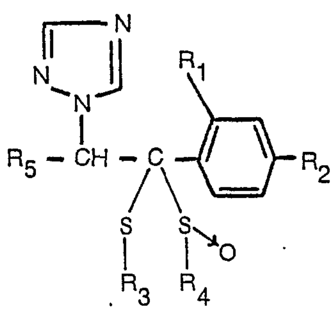 oder 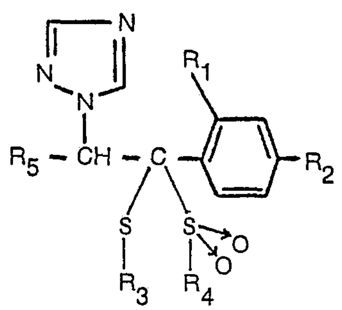 (I—c)

oxidiert wird und gewünschtenfalls von den Produkten der vorstehenden Stufen Säureadditionssalze oder Metallsalzkomplexe bereitet werden und gewünschtenfalls stereochemisch isomere Formen der Verbindung (I) hergestellt werden.

15. Verfahren zur Herstellung einer Verbindung, ausgewählt aus der aus 1-[2-(2,4-Dichlorphenyl)-1,3-dithiolan-2-yl-methyl]-1*H*-1,2,4-triazol und den Säureadditionssalzen und Metallsalzkomplexen hievon bestehenden Gruppe, gekennzeichnet durch die Thioketalisierung von 1-(2,4-Dichlorphenyl)-2-(1*H*-1,2,4-triazol-1-yl)ethanon oder eines Ketalderivats hievon mit 1,2-Ethandithiol.

16. Verfahren zur Herstellung einer Verbindung, ausgewählt aus der aus 1-[2-(2,4-Dichlorphenyl)-4-methyl-1,3-dithiolan-2-ylmethyl]-1*H*-1,2,4-triazol und den stereochemisch isomeren Formen und Säureadditionssalzen und Metallsalzkomplexen hievon bestehenden Gruppe, gekennzeichnet durch die Thioketalisierung von 1-(2,4-Dichlorphenyl)-2-(1*H*-1,2,4-triazol-1-yl)ethanon oder eines Ketalderivats hievon mit 1,2-Propandithiol.

17. Verfahren zur Herstellung einer Verbindung, ausgewählt aus der aus 1-[[2-(2,4-Dichlorphenyl)-4-ethyl-1,3-dithiolan-2-yl]methyl]-1*H*-1,2,4-triazol und den stereochemisch isomeren Formen und Säureadditionssalzen und Metallsalzkomplexen hievon bestehenden Gruppe, gekennzeichnet durch die Thioketalisierung von 1-(2,4-Dichlorphenyl)-2-(1*H*-1,2,4-triazol-1-yl)ethanon oder eines Ketalderivats hievon mit 1,2-Butandithiol.

18. Verfahren zur Herstellung einer Verbindung, ausgewählt aus der aus 1-[[2-(2,4-Dichlorphenyl)-4-propyl-1,3-dithiolan-2-yl]methyl]-1*H*-1,2,4-triazol und den stereochemisch isomeren Formen und Säureadditionssalzen und Metallsalzkomplexen hievon bestehenden Gruppe, gekennzeichnet durch die Thioketalisierung von 1-(2,4-Dichlorphenyl)-2-(1*H*-1,2,4-triazol-1-yl)ethanon oder eines Ketalderivats hievon mit 1,2-Pentandithiol.

**Revendications**

1. Composé choisi dans le groupe constitué par un dérivé de triazole de formule

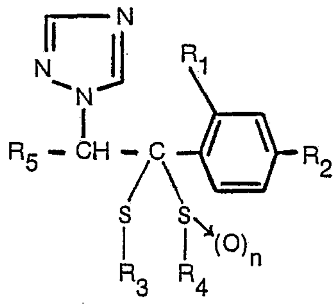

(I),

ses formes isomériques stéréochimiquement possibles, sels d'addition d'acides et complexes de sels métalliques, où:

$R_1$ et $R_2$ sont chacun indépendamment choisis dans le groupe constitué par hydrogène, halo et méthyle;

$R_3$ et $R_4$ sont chacun indépendamment choisis dans le groupe constitué par alcoyle inférieur en $C_1$ à $C_6$ et alcényle inférieur en $C_3$ à $C_4$, ou $R_3$ et $R_4$ forment, ensemble avec les atomes de soufre auxquels ils sont attachés et l'atome de carbone de pontage, un noyau à 5, 6 ou 7 chainons qui est éventuellement substitué par de 1 à 4 groupes alcoyle inférieur en $C_1$ à $C_4$;

$R_5$ est un membre choisi dans le groupe constitué par hydrogène, alcoyle inférieur en $C_1$ à $C_4$, 2-propényle et 2-propynyle; et n vaut 0, 1 ou 2.

2. Composé selon la revendication 1 où $R_1$ est un hydrogène ou un chloro et $R_2$ est un chloro.

3. Composé selon la revendication 1 où $R_1$ est un hydrogène ou un chloro, $R_2$ est un chloro, $R_3$ et $R_4$ ensemble avec les atomes de soufre auxquels ils sont attachés et l'atome de carbone de pontage forment un noyau à 5 ou 6 chainons que est éventuellement substitué par un radical alcoyle inférieur en $C_1$ à $C_3$ et $R_5$ est un hydrogène ou un alcoyle inférieur en $C_1$ à $C_4$.

4. Composé selon la revendication 1 où $R_1$ et $R_2$ sont tous deux un chloro, $R_3$ et $R_4$ avec les atomes de soufre auxquels ils sont attachés et l'atome de carbone de pontage forment un noyau à 5 chainons qui est éventuellement substitué par un groupe méthyle, éthyle ou propyle et $R_5$ est un hydrogène.

5. Composé choisi dans le groupe constitué par le 1-[[2-(2,4-dichlorophényl)-1,3-dithiolan-2-yl]méthyl]-1*H*-1,2,4-triazole et ses sels d'addition d'acides et complexes de sels métalliques.

6. Composé choisi dans le groupe constitué par le 1-[[2-(2,4-dichlorophényl)-4-méthyl-1,3-dithiolan-2-yl]méthyl]-1*H*-1,2,4-triazole et ses sels d'addition d'acides et complexes de sels métalliques.

7. Composé choisi dans le groupe constitué par le 1-[[2-(2,4-dichlorophényl)-4-éthyl-1,3-dithiolan-2-yl]méthyl]-1*H*-1,2,4-triazole et ses sels d'addition d'acides et complexes de sels métalliques.

8. Composé choisi dans le groupe constitué par le 1-[[2-(2,4-dichlorophényl)-4-propyl-1,3-dithiolan-2-yl]méthyl]-1*H*-1,2,4-triazole et ses sels d'addition d'acides et complexes de sels métalliques.

9. Composé choisi dans le groupe constitué par le 1-[1-[2-(2,4-dichlorophényl)-1,3-dithiolan-2-yl]propyl]-1*H*-1,2,4-triazole et ses sels d'addition d'acides et complexes de sels métalliques.

10. Composé choisi dans le groupe constitué par le 1-[1-[2-(2,4-dichlorophényl)-1,3-dithian-2-yl]propyl]-1*H*-1,2,4-triazole et ses sels d'addition d'acides et complexes de sels métalliques.

11. Composition antifongique comprenant des supports inertes et comme ingrédient actif und quantité antifongiquement efficace d'un composé selon l'une quelconque des revendications 1 à 10.

12. Composé selon l'une quelconque des revendications 1 à 10 ou une composition selon la revendication 11 aux fins d'application comme agent antifongique.

13. Procédé pour combattre les champignons dans lequel on met en contact lesdits champignons avec une quantité antifongiquement efficace d'un composé selon l'une quelconque des revendications 1 à 10.

14. Procédé de préparation d'un composé selon la revendication 1, caractérisé en ce qu'on soumet une cétone de formule

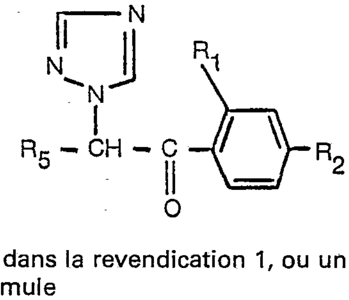

(II)

où $R_1$, $R_2$ et $R_5$ sont tels que définis dans la revendication 1, ou un de ses dérivés cétal, à une réaction de thiocétalisation avec un thiol de formule

(III) $R_3$—SH ou $R_4$—SH (IV)

ou un dithiol de formule

HS—$R_3$—$R_4$—SH (V)

où $R_3$ et $R_4$ sont tels que définis dans la revendication 1 pour obtenir un composé de formule

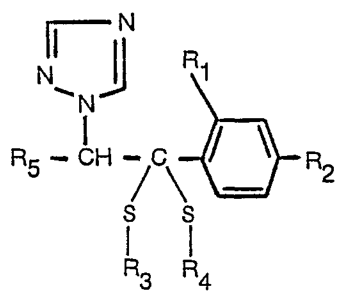

(I—a)

et éventuellement on oxyde le composé (I-a) ainsi obtenu pour obtenir un composé de formule

(I—b) 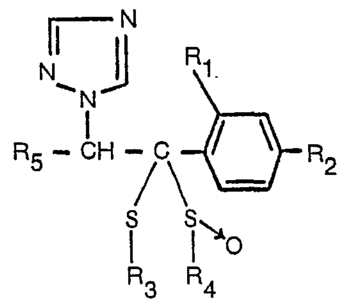 ou 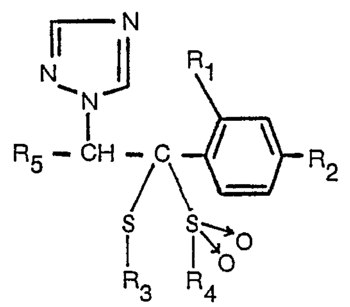 (I—c)

et si on le désire on prépare les sels d'addition d'acides ou complexes de sels métalliques des produits des étapes ci-dessus et également, si on le désire, on prépare des formes stéréochimiquement isomériques du composé (I).

15. Procédé de préparation d'un composé choisi dans le groupe constitué par le 1-[2-(2,4-dichlorophényl)-1,3-dithiolan-2-yl-méthyl]-1*H*-1,2,4-triazole et ses sels d'addition d'acides et complexes de sels métalliques, caractérisé par la thiocétalisation de la 1-(2,4-dichlorophényl)-2-(1*H*-1,2,4-triazol-1-yl)éthanone ou d'un de ses dérivés cétal avec le 1,2-éthanedithiol.

16. Procédé de préparation d'un composé choisi dans le groupe constitué par le 1-[2-(2,4-dichlorophényl)-4-méthyl-1,3-dithiolan-2-ylméthyl]-1*H*-1,2,4-triazole et ses formes stéréochimiquement isomériques et sels d'addition d'acides et complexes de sels métalliques, caractérisé par la thiocétalisation de la 1-(2,4-dichlorophényl)-2-(1*H*-1,2,4-triazol-1-yl)éthanone ou d'un de ses dérivés cétal avec le 1,2-propanedithiol.

17. Procédé de préparation d'un composé choisi dans le groupe constitué par le 1-[[2-(2,4-dichlorophényl)-4-éthyl-1,3-dithiolan-2-yl]méthyl]-1*H*-1,2,4-triazole et ses formes stéréochimiquement isomériques et sels d'addition d'acides et complexes de sels métalliques, caractérisé par la thiocétalisation de la 1-(2,4-dichlorophényl)-2-(1*H*-1,2,4-triazol-1-yl)éthanone ou d'un de ses dérivés cétal avec le 1,2-butanedithiol.

18. Procédé de préparation d'un composé choisi dans le groupe constitué par le 1-[[2-(2,4-dichlorophényl)-4-propyl-1,3-dithiolan-2-yl]méthyl]-1*H*-1,2,4-triazole et ses formes stéréochimiquement isomériques et sels d'addition d'acides et complexes de sels métalliques, caractérisé par la thiocétalisation de la 1-(2,4-dichlorophényl)-2-(1*H*-1,2,4-triazol-1-yl)éthanone ou d'un de ses dérivés cétal avec le 1,2-pentanedithiol.